Europäisches Patentamt

**European Patent Office**

Office européen des brevets

(11) Publication number: **0 233 731**
**A2**

(12) **EUROPEAN PATENT APPLICATION**

(21) Application number: **87300980.7**

(22) Date of filing: **04.02.87**

(51) Int. Cl.³: **A 61 L 2/18**
**A 01 N 37/16**

(30) Priority: **18.02.86 GB 8603960**

(43) Date of publication of application:
**26.08.87 Bulletin 87/35**

(84) Designated Contracting States:
**AT BE CH DE ES FR GB IT LI NL**

(71) Applicant: **Interox Chemicals Limited**
**Hanover House 14 Hanover Square**
**London W1R 0BE(GB)**

(72) Inventor: **Baldry, Michael Gordon Charles**
**9, Willoughby Close Old Hall**
**Warrington Cheshire WA5 5QP(GB)**

(72) Inventor: **Redmayne, William Harold**
**33, Tuson drive**
**Widnes Cheshire(GB)**

(74) Representative: **Pearce, Timothy et al,**
**Laporte Industries Limited Group Patent Department P.O.**
**Box 2 Moorfield Road**
**Widnes Cheshire WA8 0JU(GB)**

(54) **Disinfection process.**

(57) Low molecular weight monoperoxyaliphatic acids such as peracetic acid are useful as broad spectrum biocides. An even broader spectrum of activity is achieved by selecting monoperoxyaliphatic acids having a carbon chain length of 6 to 9 atoms, which act as sporicides and in a wider pH range. A preferred biocide is peroxyheptanoic acid. The process can be carried out on solid surfaces such as in place equipment, or containers or in liquid media such as process or recirculating waters.

Croydon Printing Company Ltd.

EP 0 233 731 A2

Disinfection Process

The present invention relates to disinfection and more particularly to the disinfection of surfaces or media employing a peroxygen compound as disinfectant.

There is a universal requirement for disinfectants that are effective against a broad spectrum of microoganisms. Thus, such disinfectants would be welcomed domestically for disinfecting toilets, work surfaces, sinks and drains in horticulture for disinfecting glass houses, pots and in farming for disinfecting animal or poultry houses or milking parlours in industry and especially the food and drinks industry for disinfecting of plant and apparatus in place, of cartons, canisters or containers, and process water or cooling fluid, especially if it recirculated, and in hospitals and other large-scale users of recirculating water for space heating. It has increasingly been recognised that low molecular weight monoperoxyaliphatic acids, and especially peroxyacetic acid, are broad spectrum biocides that have a wide applicability against bacteria, particularly in vegetative form, and are usable against viruses, moulds and yeasts. Accordingly there has been a significant increase in the extent of usage of peroxyacetic acid for disinfection, both as regards areas of use and amount employed. However, there is a continuing value in seeking further broad spectrum disinfectants that can either extend the conditions under which the disinfectant is effective or the microorganisms against which the disinfectant can be employed. It has been found that peroxyacetic acid is comparatively ineffective against bacterial spores and in alkaline conditions.

069xp-cs

In addition, in the course of their investigations, the inventors of the instant invention have found that there are disadvantages inherent in the use of aliphatic monoperoxyacids that have a relatively high molecular weight, typified by monoperoxydecanoic acid(perlauric acid) which means that such peracids are less effective biocides than peracetic acid in use.

Surprisingly, it has now been found that a broad spectrum biocide that preserves its activity over a wider range of conditions than does peroxyacetic acid and is more effective against spores can be obtained by selection of aliphatic monoperoxyaliphatic acids having linear carbon chain length of 6-9 atoms.

The use peracids of the fatty acid series to provide fungicidal, bactericidal, antibrowning or color restoration treatment to plant tissue has been suggested in US Patent 2 512 640, but the specification does not describe or exemplify anywhere the selected range of peroxyacids of the instant invention, nor does it suggest anywhere that such a range could show the advantageous biocidal properties found by the inventors.

Accordingly, there is provided by the present invention a process for disinfecting solid surfaces or liquid media by bringing such an infected surface or medium into contact with an effective amount of a peroxyacid of formula $R-CO_3H$ in which R represents an aliphatic hydrocarbon group having a carbon chain length of 5 to 8 atoms.

Advantageously, such peroxyacids show enhanced activity against a broad spectrum of microorganisms including spores and under alkaline conditions, whilst retaining effective activity under acid or neutral conditions.

It is often convenient to employ the invention disinfectant in the form of an aqueous solution, normally at a concentration of at least $4 \times 10^{-4}M$ of peroxyacid and particularly selected in the range of $1 \times 10^{-3}$ to $1 \times 10^{-2}M$. A saturated solution can be used. Particularly effective results have been obtained in the range of $2 \times 10^{-3}M$ to

$5 \times 10^{-3}$M. Whilst the solution can be employed at its natural, mildly acidic pH, it can be adjusted to be more acidic down to pH3, suitably by the addition of a mineral acid or more alkali suitably up to pH 11, for example, by addition of an alkali, and particularly an alkali metal-based alkali such as sodium hydroxide, or sodium carbonate. One method of obtaining such solutions is by the introduction of solid preformed peroxyacid into solution.

The disinfectant can suitably be any of the linear monoperoxy aliphatic acids containing 6 to 9 carbon atoms, including specifically peroxyhexanoic acid, peroxyheptanoic acid, peroxyoctanoic acid and peroxynonanoic acid, and preferably is peroxyheptanoic acid in view of its manifest activity profile. Alternatively, the aliphatic chain can be non-linear, the compound preferably having a molecular weight of not more than 174. A suitable example of a non-linear peroxyacid is 3,5,5-trimethylperoxyhexanoic acid.

The process of disinfection can be carried by simply bringing the disinfectant into contact with the surface or media to be disinfected and maintaining contact for a brief period. Very satisfactory results have been obtained using a contact period of 5 minutes. Some disinfection will occur with shorter contact periods, such as desirably from 30 seconds upwards, and often at least 1 minute. Longer contact periods can be tolerated and are at the discretion of the user. By way of example, the surface or media can be permitted to remain in contact with the peroxyacid disinfectant until it has lost all its peroxyacid active oxygen. Accordingly, the contact period may last several hours or even days, when conditions so permit. The contact may be by a single shot addition of the peroxyacid or by periodic or slow constant addition such as to a bath or recirculating liquor. It will be recognised that if the medium to be disinfected is itself an aqueous medium, it can be treated by direct introduction of the selected peroxyacids whilst maintaining the solution pH within the range given previously herein.

069xp-cs

4

The invention process can be carried out over a wider range of temperatures, for example from 0° to 100°C. Accordingly, the process can in many instances be effected without any change to the temperature of the substrate or media before treatment. Likewise the process can be readily carried out under the usual range of ambient temperatures encompassing both winter and summer conditions and conditions both indoors and out of doors, such as often in the range 10 to 30°C.

The methods of applying the instant invention process to surfaces and media are in essence the same as have hitherto been suggested for the application of peroxyacetic acid for the same purpose. Thus, for example, for liquid media the disinfectant can be directly introduced therein, and permitted to dissolve, thereby controlling the growth of microorganisms in recirculating process or heating waters or in discharges to public water courses. For the treatment of fixed apparatus or plant or work surfaces, walls or the like, a solution of the disinfectant can be sprayed or poured onto the surface to be disinfected and small articles such as containers or canisters can be immersed or sprayed.

Advantageously, it has also been found that not only are the disinfectants very effect against a broad spectrum of mono-organisms but in addition they tend to smell less repulsivey than peroxyacetic acid does. To that extent, therefore, they are more acceptable to users.

Having described the invention briefly, the following examples are given in greater detail to demonstrate the invention process.

The microorganisms used in the tests were as follows:-

Ec    Escherichia coli NCTC 8196 (faecal organism)

Pa    Pseudomonas aeruginosa ATCC 15442 (Urinary tract pathogen)

Sa    Staphylococcus aureus ATCC 6538 (skin organism)

Sf    Streptococcus faecalis ATCC 10541 (faecal organism)

5

Ca   Candida albicans ATCC 10231 (vaginal pathogen)

Sc   Saccharaomyces cerevisiae ATCC 9763

Bs   Bacillus subtilis NCTC 10452 (spore former)

Each test was carried out by a standardised suspension method. Sub cultures of the stock cultures were cultured in an appropriate medium, separated therefrom by centrifugation washed with sodium sulphate solution and then resuspended in fresh sodium sulphate solution at a concentration of between $10^8$ and $3\times10^8$ cfu ml$^{-1}$ for asporogenous bacteria and between $10^7$ and $3\times10^7$ cfu ml$^{-1}$ for yeasts and B. subtilis determined photometrically by comparing the measurement with standard suspension concentrations. A solution of the biocide at twice its tested concentration was made in demineralised water within 60 minutes prior to the test and a 5.0ml portion transferred to a sterile test tube and diluted with 40.ml demineralised water and 1.0ml of microbial suspension. Thus, in each test a solution of one of the microorganisms was dosed into the liquid biocidal solution to provide an initial concentration of about $10^6$ to $10^7$ cfu ml$^{-1}$. The abbreviation "cfu" indicates "colony-forming units".

At the end of the test period of 5 minutes, the solutions containing biocide were neutralised as to biocidal activity by the addition of a CT neutraliser except for the combinations of peroxyheptanoic acid and Bs/Sc and peroxynonanoic acid and Pa/Sa/Sc/Bs when TTLC neutraliser was used. CT neutraliser consisted of a mixture by volume of 1 part of a catalase solution of Sigma C-10 catalase 2.5g in 1 litre demineralised water sterilised by membrane filtration, pore size $4.5\times10^{-7}$m and 9 parts of a solution of sodium thiosulphate pentahydrate (50g) in 1 litre of demineralised water sterilised at 121$^\circ$C. The TTLC neutraliser was the same as the CT neutraliser except that the thiosulphate was dissolved in a mixture of a nonionic surfactant (TWEEN 80) 30ml and demineralised water 970ml, and additionally contained Sigma P-5638 (L-phosphatidylcholine) 20g.

069xp-cs

6

The concentrations of viable cells in the innocula are obtained by the method of serial dilution in Ringer solution and followed by the conventional p. plate methods with incubation plates at 37°C (30°C for S. cerevisiae for 48 hours). The mean number of colonies is then counted. Identical incubation conditions are used for the neutralised solutions.

The results of the tests are presented in the Table below, in which PAA represents the comparison peroxyacetic acid, PLA the comparison peroxylauric acid (peroxydodecanoic acid) PHA peroxyheptanoic acid and PNA peroxynonanoic acid. The PLA comparison peroxyacid could be introduced at the concentration of only $1.16 \times 10^{-3}$ moles per litre rather than the higher concentrations of the other peroxyacids because it was less soluble than them, and even then it was not completely dissolved. The extent to which the microorganism population was reduced is indicated by the figures given in the Table, which are expressed on a logarithmic scale (base 10) or by +++ showing that no microorganisms survived or --- showing that no significant reduction in population had occured.

7

## The Table

| Micro-Organism | Biocide | Molar conc. $\times 10^{-3}$ | Population Reduction pH5 | pH7 | pH9 |
|---|---|---|---|---|---|
| Vegetative Bacteria | | | | | |
| Ec | PAA | 2 | +++ | +++ | +++ |
| | PHA | 2 | +++ | +++ | +++ |
| | PNA | 2 | +++ | +++ | +++ |
| | PLA | 1.16* | --- | --- | 6 |
| Pa | PAA | 2 | +++ | +++ | 5 |
| | | 4 | +++ | +++ | 5 |
| | PHA | 2 | 6 | +++ | +++ |
| | | 4 | +++ | +++ | +++ |
| | PNA | 2 | +++ | +++ | 6 |
| | | 2.5 | +++ | +++ | +++ |
| | PLA | 1.16* | --- | --- | --- |
| Sa | PAA | 2 | +++ | +++ | +++ |
| | PHA | 2 | +++ | +++ | +++ |
| | PNA | 2 | +++ | 6 | +++ |
| | | 2.5 | +++ | +++ | +++ |
| | PLA | 1.16* | 4.3 | 5 | 5 |
| Sf | PAA | 2 | +++ | +++ | --- |
| | | 4 | +++ | +++ | +++ |
| | PHA | 2 | +++ | +++ | +++ |
| | PNA | 2 | +++ | +++ | +++ |
| | PLA | 1.16* | 6 | 4.6 | 4.3 |

069xp-cs

8

| Micro-Organism | Biocide | Molar conc. x10$^{-3}$ | Population Reduction | | |
|---|---|---|---|---|---|
| | | | pH5 | pH7 | pH9 |
| Yeasts | | | | | |
| Ca | PAA | 2 | +++ | --- | --- |
| | | 4 | +++ | +++ | --- |
| | PHA | 2 | +++ | +++ | +++ |
| | PNA | 2 | +++ | +++ | +++ |
| | PLA | 1.16* | 4 | --- | --- |
| Sc | PAA | 2 | +++ | +++ | 4 |
| | | 4 | +++ | +++ | 4 |
| | PHA | 2 | +++ | +++ | +++ |
| | PNA | 2 | +++ | +++ | +++ |
| | PLA | 1.16* | 4 | 4.1 | 3.7 |
| Bacterial Spore | | | | | |
| Bs | PAA | 4 | --- | --- | --- |
| | PHA | 2 | +++ | +++ | --- |
| | PNA | 2 | 5 | --- | --- |
| | | 2.5 | 5 | 3.4 | --- |
| | PLA | 1.16* | --- | --- | --- |

From the Table above, it can be seen that at the same molar concentration the peroxyacids PHA and PNA performed better than did the comparison biocides, PAA and PLA as regards the range of microorganisms and pH conditions under which they were able to be used. Not only can the invention biocides be used against gram positive and gram negative bacteria but also are more effective against yeasts and spores.

CLAIMS

1. A process for disinfecting solid surfaces or liquid media by bringing such an infected surface or medium into contact with an effective amount of a peroxyacid characterised in that the peroxyacid has the formula $R-CO_3H$ in which R represents an aliphatic hydrocarbon group having a carbon chain length of 5 to 8 atoms.

2. A process according to claim 1 characterised in that the peroxyacid is a linear $C_6$-$C_{10}$ monoperoxyacid.

3. A process according to claim 2 characterised in that the peroxyacid is peroxyheptanoic acid.

4. A process according to any preceding claim characterised in that the peroxyacid is employed in an aqueous solution at a concentration of $10^{-3}M$ to $10^{-2}M$.

5. A process according to any preceding claim characterised in that the peroxyacid is employed at a pH of from 3 to 11.

6. A process according to any preceding claim characterised in that the peroxyacid is brought into contact with spores.

7. A process for disinfection employing any novel feature or combination of features described herein.